Europäisches Patentamt

(19) European Patent Office

Office européen des brevets

(11) Publication number: **0 444 855 A1**

# (12) EUROPEAN PATENT APPLICATION

(21) Application number: 91301504.6

(51) Int. Cl.$^5$: **C07C 217/20, A61K 31/135**

(22) Date of filing: 26.02.91

(30) Priority: 28.02.90 US 486478
19.11.90 US 615201

(43) Date of publication of application:
04.09.91 Bulletin 91/36

(84) Designated Contracting States:
AT BE CH DE DK ES FR GB GR IT LI LU NL SE

(71) Applicant: ELI LILLY AND COMPANY
Lilly Corporate Center
Indianapolis Indiana 46285 (US)

(72) Inventor: Fuller, Ray Ward
1008 Hudson Bay Drive
Greenwood, Indiana 46142 (US)

Inventor: Mitchell, David
6099 Pillory Drive
Indianapolis, Indiana 46254 (US)
Inventor: Robertson, David Wayne
4290 Hunters Ridge Lane
Greenwood, Indiana 46142 (US)
Inventor: Stephenson, Gregory Alan
Purdue University, Hawkins Grad House,
Room 1226
West Lafayette, Indiana 47906 (US)
Inventor: Wong, David Taiwai
1640 Ridge Hill Lane
Indianapolis, Indiana 46217 (US)

(74) Representative: Hudson, Christopher Mark et
al
Erl Wood Manor
Windlesham Surrey GU20 6PH (GB)

(54) Improvements in and relating to (S)-norfluoxetine.

(57) The present invention provides novel crystalline forms of (S)-norfluoxetine hydrochloride.

EP 0 444 855 A1

# IMPROVEMENTS IN AND RELATING TO (S)-NORFLUOXETINE

This invention provides novel crystalline forms of (S)-norfluoxetine hydrochloride.

During the past decade, the relationship between monoamine uptake and a variety of diseases and conditions has been appreciated and investigated. For example, the hydrochloride salt of fluoxetine (dl-N-methyl-3-[4-(trifluoromethyl)phenoxy]-3-phenylpropyl-amine) is a selective serotonin (5-hydroxytryptamine) uptake inhibitor. Fluoxetine hydrochloride is marketed in the U.S. under the trademark PROZAC® for the treatment of depression. This compound is among many taught in U.S. Patents Number 4,018,895, 4,194,009, and 4,314,081 as being potent, selective blockers of serotonin uptake.

Fluoxetine is a racemate of the two enantiomeric forms. The biological and pharmacological activity of each enantiomer has been found to be essentially the same; see, Robertson et al., J. Med. Chem., 31, 1412 (1988) and references cited therein.

Norfluoxetine [3-(4-trifluoromethylphenoxy)-3-phenylpropylamine] is a metabolite of fluoxetine and is known to block monoamine uptake, especially serotonin. See U.S. Patent Number 4,313,896. Norfluoxetine had only been evaluated as the racemate, and since it is a metabolite of fluoxetine, it was believed that this compound contributes in part to the biological activity seen upon administration of fluoxetine. Since the eudismic ratio for fluoxetine, i.e., the ratio of affinities or activities of its two enantiomers, is approximately unity, conventional wisdom would have suggested that the individual enantiomers of norfluoxetine would similarly have equivalent activities. Surprisingly, in accordance with the invention, it has now been discovered that the (S)-enantiomer of norfluoxetine is substantially more active than its (R)-optical antipode.

(S)-Norfluoxetine can be prepared by any of a number of methods generally known in the art. For example, there are several methods provided in the literature for making the racemate of norfluoxetine, see U.S. Patent 4,313,896. The racemate of norfluoxetine in turn can be resolved into its (S) and (R) components by standard methods. In particular, norfluoxetine can be reacted with an enantiomerically pure chiral derivatizing agent, resolved on the basis of the different physicochemical properties of the diastereomeric derivatives, and then converted to the two separate enantiomers of norfluoxetine. One particularly preferred method of accomplishing this derivatization is analogous to that described in Robertson et al., J. Med. Chem., 31, 1412 (1988), wherein fluoxetine was reacted with an optically active form of 1-(1-naphthyl)ethyl isocyanate to form a urea derivative of fluoxetine. A similar mixture of norfluoxetine diastereomeric ureas can be separated through high pressure liquid chromatography into the individual diastereomers. Each individual diastereomer, in turn, can then be hydrolyzed to the individual enantiomers of norfluoxetine.

A preferred method of preparing (S)-norfluoxetine is similar to that labeled Scheme I in the Robertson et al. reference. (S)-(-)-3-Chloro-1-phenylpropanol (II) is either commercially available or can be prepared by chiral reduction of 3-chloropropiophenone. II can be transformed into (S)-3-amino-1-phenylpropanol (III). Although a number of routes to convert the chloride intermediate to the amino compound are available, the preferred method is the transformation of the chloride to an intermediate N-substituted phthalimide which can be transformed to the desired primary amino intermediate III. This reaction sequence is a Gabriel synthesis wherein the potassium salt of phthalimide is reacted with (S)-(-)-3-chloro-1-phenylpropanol, preferably in the presence of a nonreactive solvent such as dimethylformamide or especially dimethylsulfoxide, to prepare the (S)-3-phthalimido-1-phenylpropanol intermediate. The phthalimido intermediate may be hydrolyzed to provide the desired amino intermediate III. However, to prevent the possible racemization of the intermediate, the phthalimide intermediate is preferably treated with hydrazine in a nonreactive solvent such as ethanol to provide the desired (S)-3-amino-1-phenylpropanol intermediate III. This latter compound can then be reacted with sodium hydride in dimethylacetamide or some other nonreactive solvent, preferably dimethylsulfoxide, to generate the alkoxide which, upon treatment with 4-chloro-or 4-fluoro-benzotrifluoride, leads to a facile nucleophilic aromatic substitution to provide (S)-norfluoxetine.

Alternatively, commercially available (R)-3-phenyloxiranemethanol ((2R,3R)-(+)-3-phenylglycidol) can be treated with a reducing agent such as sodium bis(2-methoxyethoxy)aluminum hydride in a non-reactive solvent such as dimethoxyethane to provide (S)-1-phenyl-1,3-propanediol. The primary alcohol group of this diol intermediate is then converted into a good leaving group which can be displaced with ammonia. For example (S)-1-phenyl-1,3-propanediol is treated with a non-reactive base in an inert solvent, such as the use of triethylamine in tetrahydrofuran or dichloromethane. Treatment with a sulfonyl chloride, such as methanesulfonyl chloride, p-toluenesulfonyl chloride, p-chlorophenylsulfonyl chloride, or preferably p-bromophenylsulfonyl chloride, provides the corresponding sulfonate ester (eg, the mesylate, tosylate, p-chlorophenylsulfonate, or p-bromophenylsulfonate, respectively). When any of these sulfonate esters are treated with ammonia, for example, gaseous ammonia dissolved in an alcohol, such as methanol, under pressure, for example at 60 p.s.i., provides the corresponding amine sulfonate salt which can be converted to III upon treatment with base. This sequence is preferred for large scale preparation.

A less direct way of preparing III involves taking a sulfonate ester (as described in the preceding paragraph) of commercially available (R)-1-phenyl-1,2-ethanediol, protecting the remaining alcohol with, for example, a silyl group, such as reacting the alcohol with t-butyldimethylsilyl chloride in the presence of a non-reactive base, such as imidazole, in an inert solvent such as dimethylformamide. This protected sulfonate ester can then be reacted with cyanide, such as with potassium or sodium cyanide, in a non-reactive solvent, such as dimethylformamide or dimethylsulfoxide, at temperature of about 50-100°C, to give silyl protected (R)-3-phenyl-3-hydroxypropionitrile, which can be reduced (eg, a borane or aluminum hydride reagent, particularly borane-tetrahydrofuran complex in tetrahydrofuran) and hydrolyzed (eg, treatment with 3 N hydrochloric acid) to give III. This procedure is particularly useful for preparing radiolabelled III, such as by using $^{14}$C-labelled sodium cyanide.

It has been discovered that the hydrochloride salt is particularly well adapted for pharmaceutical use. The following example further illustrates the preparation of (S)-norfluoxetine hydrochloride.

## Example 1

(S)-Norfluoxetine Hydrochloride

A. Preparation of (S)-3-phthalimido-1-phenylpropanol.

To a solution of 470 mg of (S)-(-)-3-chloro-1-phenylpropanol in 4 ml of dimethylformamide were added 612 mg of potassium phthalimide in 4 ml of dimethylformamide. The mixture was heated at 100°C for 6 hours, allowed to cool to room temperature, and stirred overnight. The mixture was filtered, the filtrate diluted with water, and the solution extracted with ethyl acetate. The organic layer was washed once with water, once with 0.2 N sodium hydroxide, once again with water, and once with a saturated solution of sodium chloride, dried over sodium sulfate, and concentrated in vacuo to provide 730 mg of an opaque oil that solidified. Crystallization from ethyl acetate/hexanes provided 350 mg of the title intermediate as a white powder, m.p. 80-82.5°C.

Analysis for $C_{17}H_{15}NO_3$:

Calc.:     C, 72.58; H, 5.38; N, 4.98;

Found:     C, 72.57; H, 5.40; N, 4.96.

B. Preparation of (S)-3-amino-1-phenyl-1-propanol.

To a solution of 4.04 g of (S)-3-phthalimido-1-phenyl-1-propanol in 100 ml of ethanol was added 2.5 ml of anhydrous hydrazine. The mixture was heated to reflux under a nitrogen atmosphere for 3.5 hours, cooled to room temperature, and allowed to stir overnight. The resulting precipitate was removed by filtration and the filtrate concentrated in vacuo. The resulting oil was treated with diethyl ether and 25 ml of 5 N sodium hydroxide. The layers were separated, and the organic layer was dried over sodium sulfate and concentrated in vacuo to provide 1.92 g of an opaque oil. Two hundred milligrams of this oil was treated with oxalic acid in ethyl acetate and crystallized from ethyl acetate/methanol to provide 210 mg of the title intermediate as the oxalate salt, m.p. 161-162°C.

Analysis of the oxalate salt: $C_{11}H_{15}NO_5$:

Calc.:     C, 54.77; H, 6.27; N, 5.81;

Found:     C, 54.96; H, 6.15; N, 5.79.

C. Preparation of (S)-norfluoxetine hydrochloride.

To a slurry of 484 mg of 60% sodium hydride in oil in 10 ml of dimethylacetamide were added 1.74 g of (S)-3-amino-1-phenyl-1-propanol in 40 ml of dimethylacetamide. The mixture was heated at 70°C for 10 minutes. 4-Fluorobenzotrifluoride (1.54 ml) was added to the reaction mixture and the solution heated at 100°C for 3 hours. The mixture was poured into ice water and extracted into diethyl ether. The organic extract was washed three times with water, once with a saturated sodium chloride solution, dried over sodium sulfate and concentrated in vacuo to provide 2.96 g of a yellow oil. The oil was purified by high pressure liquid chromatography over silica gel eluting with a gradient of methylene chloride to 10% methanol in methylene chloride to which 0.5% of ammonium hydroxide had been added. The desired fractions were combined and concentrated in vacuo to yield 1.5 g of the title product (base) as an amber oil.

A solution of 229 mg (S)-norfluoxetine in 20 ml of diethyl ether was treated with hydrogen chloride gas. After 30 ml of hexanes were added, the solution was placed in the freezer. The resulting solid was recovered by filtration affording 162 mg of the title product, m.p. 128-130°C.

Analysis for $C_{16}H_{16}F_3N \cdot HCl$:

Calc.:     C, 57.93; H, 5.17; N, 4.22;

Found:     C, 57.86; H, 4.94; N, 4.15.

As can be seen from the above, the aforementioned (S)-norfluoxetine hydrochloride was prepared by dissolving the free base material in diethyl ether, bubbling in hydrogen chloride gas, adding hexanes, and cooling. X-ray powder diffraction analysis determined that such material was either amorphous or a poorly defined mixture of crystalline forms and amorphous material. The use of concentrated hydrochloric acid in ether and attempting to crystallize from diethyl ether/cyclohexane provided the same result. This material was therefore unsuitable for use in pharmaceutical preparations much as tablets or capsules since it tended to be hygroscopic and thus lacked the necessary stability. This invention provides stable crystalline forms of (S)-norfluoxetine hydrochloride suitable for direct use in pharmaceutical formulations such as tablets and capsules. The following example illustrates the preparation of a novel crystalline form of (S)-norfluoxetine hydrochloride which will be desirated hereinafter as "Form 1".

Example 2.

5.1 g of (S)-norfluoxetine hydrochloride were dissolved in 15 ml of wet diethyl ether (diethyl ether which was shaken with 200 ml of water and the layers separated before use). Petroleum ether (13 ml) was added, and the solution allowed to stand at room temperature overnight. The resulting solid was recovered by filtration, washed with petroleum ether, and dried to provide 3.33 g of crystalline (S)-norfluoxetine hydrochloride Form 1, m.p. 130-131.5°C. A representative X-ray powder diffraction pattern using a diffractometer with a graphite monochromator (Cu/K alpha radiation wavelength = 1.5418 Angstroms) for this material is given below:

## (S)-Norfluoxetine Hydrochloride Form 1

| Interplanar Spacing d (Å) | Intensity Ratio $I/I_o$ |
|---|---|
| 16.83 | 0.15 |
| 11.89 | 0.19 |
| 10.63 | 0.03 |
| 7.51 | 0.10 |
| 6.05 | 0.03 |
| 5.95 | 0.07 |
| 5.69 | 0.30 |
| 5.60 | 0.02 |
| 5.39 | 0.18 |
| 5.30 | 0.03 |
| 5.13 | 0.10 |
| 4.91 | 0.43 |
| 4.76 | 0.05 |
| 4.67 | 0.05 |
| 4.54 | 0.13 |
| 4.38 | 0.12 |
| 4.20 | 1.00 |
| 4.11 | 0.13 |
| 4.08 | 0.20 |
| 3.96 | 0.10 |
| 3.89 | 0.05 |
| 3.76 | 0.56 |

| Interplanar Spacing d (Å) | Intensity Ratio $I/I_o$ |
|---|---|
| 3.69 | 0.05 |
| 3.53 | 0.06 |
| 3.45 | 0.18 |
| 3.36 | 0.21 |
| 3.30 | 0.05 |
| 3.19 | 0.04 |
| 3.12 | 0.03 |
| 3.06 | 0.04 |
| 2.94 | 0.08 |
| 2.77 | 0.05 |
| 2.61 | 0.04 |
| 2.57 | 0.04 |
| 2.49 | 0.01 |
| 2.36 | 0.04 |
| 2.13 | 0.03 |
| 2.06 | 0.02 |
| 1.62 | 0.01 |

Form 1 can be prepared by recrystallizing other forms of (S)-norfluoxetine hydrochloride or poorly defined or amorphous hydrochloride salt from a mixture of a wet ether (i.e., an ether such as diethyl ether or preferably t-butyl methyl ether which is shaken with water and the layers separated before use) and either petroleum ether or heptane. Form 1 is also, and preferably, obtained by crystallization of non-Form 1 (S)-norfluoxetine hydrochloride from no more than 15% (v/v) ethyl acetate in heptane.

Form 1 is also formed from Form 2 on prolonged standing. Thus, this invention also provides for (S)-norfluoxetine hydrochloride Form 2 which can be used as an intermediate in preparing Form 1. Crystalline (S)-norfluoxetine hydrochloride designated Form 2 is obtained in the same manner as described above when the solvent combination tetrahydrofuran(THF)/heptane or ethyl acetate/cyclohexane is used to prepare or recrystallize the product. Although the melting point of Form 2 appears to be 130°C, the melt which is being observed is actually that of Form 1. During the melting point determination, there is a solid state transition of Form 2 into Form 1. The transition is fast at elevated temperatures and occurs more slowly at room temperature. The following Example illustrates the preparation of Form 2.

Example 3

Four grams of (S)-norfluoxetine hydrochloride were dissolved in 16 ml of THF. Sixty ml of heptane were added, the solution heated to reflux, and additional THF added to homogeneity. The solution was cooled and allowed to stand at room temperature. The resulting product (2.5 g) was recovered as white needles. After allowing to stand for several months, the product was identified as Form 2 material. A representative X-ray powder diffraction pattern for Form 2 is given below:

### (S)-Norfluoxetine Hydrochloride Form 2

| Interplanar Spacing d (Å) | Intensity Ratio $I/I_o$ |
|---|---|
| 17.67 | 0.68 |
| 15.65 | 0.23 |
| 12.04 | 0.14 |
| 11.27 | 0.39 |
| 8.86 | 0.12 |
| 7.47 | 0.13 |
| 7.23 | 0.34 |
| 6.01 | 0.38 |

| Interplanar Spacing | Intensity Ratio |
|:---:|:---:|
| d (Å) | $I/I_o$ |
| 5.70 | 0.27 |
| 5.39 | 0.47 |
| 5.22 | 0.29 |
| 4.91 | 0.69 |
| 4.84 | 0.43 |
| 4.55 | 0.22 |
| 4.32 | 1.00 |
| 4.20 | 0.45 |
| 3.94 | 0.96 |
| 3.85 | 0.68 |
| 3.76 | 0.22 |
| 3.71 | 0.20 |
| 3.53 | 0.87 |
| 3.45 | 0.30 |
| 3.30 | 0.33 |
| 3.14 | 0.16 |
| 3.07 | 0.31 |
| 2.94 | 0.22 |
| 2.32 | 0.19 |
| 2.20 | 0.13 |
| 2.02 | 0.02 |

A third form of (S)-norfluoxetine hydrochloride, designated as Form 3, is produced when prepared or crystallized from toluene, toluene/heptane, or at least 15% (v/v) ethyl acetate in heptane. The following Example illustrates the preparation of this polymorph.

Example 4

40 Grams of (S)-norfluoxetine hydrochloride were dissolved in 100 mL of ethyl acetate. The solution was heated to 75-80°C. Heptane (400 mL) was heated to 90°C and added to the (S)-norfluoxetine hydrochloride-/ethyl acetate solution. The solution mixture was allowed to cool and the resulting white crystals were recovered by filtration and washed with fresh solvent mixture to afford the Form 3 product in 68% yield. Form 3 has the following representative X-ray powder diffraction pattern:

6

## (S)-Norfluoxetine Hydrochloride Form 3

| Interplanar Spacing | Intensity Ratio |
|---|---|
| d (A) | I/I$_o$ |
| 15.49 | 0.36 |
| 14.28 | 0.01 |
| 11.36 | 0.03 |
| 9.25 | 0.03 |
| 8.79 | 0.09 |
| 7.18 | 0.10 |
| 6.70 | 0.02 |
| 6.04 | 0.23 |
| 5.92 | 0.10 |
| 5.84 | 0.05 |
| 5.68 | 0.36 |
| 5.58 | 0.08 |
| 5.49 | 0.11 |
| 5.26 | 0.08 |
| 5.19 | 0.14 |
| 4.89 | 0.12 |
| 4.79 | 0.12 |
| 4.64 | 1.00 |
| 4.57 | 0.71 |
| 4.26 | 0.03 |
| 4.17 | 0.14 |
| 4.04 | 0.06 |
| 3.84 | 0.17 |
| 3.78 | 0.22 |
| 3.76 | 0.35 |
| 3.68 | 0.20 |
| 3.60 | 0.35 |
| 3.46 | 0.10 |
| 3.35 | 0.11 |

## (S)-Norfluoxetine Hydrochloride Form 3

| Interplanar Spacing | Intensity Ratio |
|---|---|
| d (A) | I/I$_o$ |
| 3.30 | 0.10 |
| 3.25 | 0.04 |
| 3.10 | 0.04 |
| 3.02 | 0.03 |
| 2.97 | 0.04 |
| 2.94 | 0.05 |
| 2.84 | 0.08 |
| 2.60 | 0.04 |

As will be appreciated by these skilled in the art, the precise values of interplanar spacing and intensity ratios may vary slightly depending upon compound or crystalline purity, the instrument used to make such an analysis, or operator variables. This comment is particularly relevant to the Intensity Ratio ($I/I_o$) where large variations are to be expected.

This invention covers each of the polymorphs having substantially the same interplanar spacings as noted above when determined by X-ray powder diffraction using an interferometer. The polymorphs produced by the procedures described above will be substantially free from contamination of the amorphous form.

(S)-Norfluoxetine hydrochloride Forms 1 and 3 are useful for inhibiting the uptake of serotonin.

Therefore, another aspect of the present invention is a method for inhibiting serotonin uptake in mammals which comprises administering to a mammal requiring increased neurotransmission of serotonin a pharmaceutically effective amount of (S)-norfluoxetine hydrochloride Form 1 or 3.

The term "pharmaceutically effective amount", as used herein, represents an amount of (S)-norfluoxetine hydrochloride Form 1 or 3 which is capable of inhibiting serotonin uptake. The particular dose of compound administered according to this invention will, of course, be determined by the particular circumstances surrounding the case, including the route of administration, the particular condition being treated, and similar considerations. (S)-Norfluoxetine hydrochloride Form 1 or 3 can be administered by a variety of routes including the oral, rectal, transdermal, subcutaneous, intravenous, intramuscular or intranasal routes. A typical daily dose will contain from about 0.01 mg/kg to about 20 mg/kg of (S)-norfluoxetine hydrochloride Form 1 or 3. Preferred daily doses will be about 0.05 to about 10 mg/kg, ideally about 0.1 to about 5 mg/kg.

(S)-Norfluoxetine hydrochloride Forms 1 and 3 have the ability to treat a variety of disorders in mammals influenced by serotonergic systems such as obesity, bulimia, obsessive-compulsive disorders, depression, aggression, alcoholism, pain, pre-menstrual syndrome (PMS), loss of memory, anxiety, panic attack, smoking, symptoms associated with nicotine withdrawal, sleep disorders such as narcolepsy or sleep apnea, urinary incontinence, substance abuse (eg, cocaine, heroin, amphetamines, etc.), dementia, emotional disturbance associated with Alzheimer's Disease, and migraine. The compound can be used as an aid in increasing the rate of recanalization following thrombolytic or angioplasty therapy, and can be used to prevent restenosis or vasospasm following thrombolysis or angioplasty therapy. (S)-Norfluoxetine hydrochloride Forms 1 and 3 also have little effect on metabolism of concurrently administered drugs such as barbiturates or tricyclic antidepressants, unlike fluoxetine. (S)-Norfluoxetine hydrochloride Forms 1 and 3 are relatively non-toxic and has an excellent therapeutic index. Therefore, the present invention also provides methods of treating the above disorders at rates set forth above for inhibiting serotonin uptake in mammals.

The following experiment was conducted to demonstrate the ability of (S)-norfluoxetine to inhibit the uptake of serotonin as compared with its related enantiomer, racemate, and comparable fluoxetine analogs. This general procedure is set forth by Wong et al., in Drug Development Research 6:397-403 (1985).

Male Sprague-Dawley rats (110-150 g) from Harlan Industries (Cumberland, IN) were fed a Purina Chow ad libitum for at least 3 days before being used in the studies. Rats were killed by decapitation. Whole brains were removed and dissected. Cerebral cortex was homogenized in 9 volumes of a medium containing 0.32 M sucrose and 10 mM glucose. Crude synaptosomal preparations were isolated after differential centrifugation at 1,000 g for 10 min. and 17,000 g for 28 min. The final pellets were suspended in the same medium and kept in ice until use within the same day.

Synaptosomal uptake of $^3$H-serotonin ($^3$H-5-hydroxytryptamine, $^3$H-5HT) was determined as follows. Cortical synaptosomes (equivalent to 1 mg of protein) were incubated at 37°C for 5 min in 1 ml of Krebs-bicarbonate medium containing also 10 mM glucose, 0.1 mM iproniazid, 1 mM ascorbic acid, 0.17 mM EDTA, 50 nM $^3$H-5HT, and appropriate concentrations of test compound. The reaction mixture was immediately diluted with 2 ml of ice-chilled Krebs-bicarbonate buffer and filtered under vacuum with a cell harvester (Brandel, Gaithersburg, MD). Filters were rinsed twice with approximately 5 ml of ice-chilled 0.9% saline and were transferred to a counting vial containing 10 ml of scintillation fluid (PCS, Amersham, Arlington Heights, IL). Radioactivity was measured by a liquid scintillation spectrophotometer. Accumulation of $^3$H-5HT at 4°C represented the background and was subtracted from all samples.

The results of the evaluation of (S)-nor-fluoxetine and related compounds from two side-by-side experiments are set forth below in Table I. In the Table, column 1 identifies the compound evaluated, and column 2 provides the nanomolar (nM) concentration of the test compound needed to inhibit 50% of serotonin (5HT) uptake and is indicated in the Table as the $IC_{50}$. The first experiment employed an older lot of $^3$H-5HT whereas the second experiment employed a new lot of $^3$H-5HT.

## Table I

### INHIBITION OF 5HT UPTAKE IN VITRO

| Compound | 5HT $IC_{50}$ (nM) | |
|---|---|---|
| | Expt. 1 | Expt. 2 |
| (R,S)-norfluoxetine | 202 | 55.8 |
| (R)-norfluoxetine | 1051 | 484.1 |
| (S)-norfluoxetine | 69 | 29.8 |
| (R,S)-fluoxetine | 79 | 34.4 |
| (R)-fluoxetine | 127 | 39.7 |
| (S)-fluoxetine | 93 | 25.0 |

The compound of the present invention is preferably formulated prior to administration. Therefore, yet another embodiment of the present invention is a pharmaceutical formulation comprising (S)-norfluoxetine hydrochloride Form 1 and one or more pharmaceutically acceptable carriers, diluents or excipients therefor.

The present pharmaceutical formulations are prepared by known procedures using well known and readily available ingredients. In making the compositions of the present invention, the active ingredient will usually be mixed with a carrier, or diluted by a carrier, or enclosed within a carrier which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), ointments containing, for example, up to 10% by weight of the active compound, soft and hard gelatin capsules, suppositories, sterile injectable solutions and sterile packaged powders.

Some examples of suitable carriers, excipients, and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyland propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient by employing procedures well known in the art.

The compositions are preferably formulated in a unit dosage form, each dosage containing from about 5 to about 500 mg, more usually about 25 to about 300 mg, of the active ingredient. The term "unit dosage form" refers to physically discrete units suitable as unitary dosages for human subjects and other mammals, each unit containing a predetermined quantity of active material calculated to produce the desired therapeutic effect, in association with a suitable pharmaceutical carrier.

The following formulation examples are illustrative only and are not intended to limit the scope of the invention in any way.

### Formulation 1

Hard gelatin capsules are prepared using the following ingredients:

|                                               | Quantity (mg/capsule) |
| --------------------------------------------- | --------------------- |
| (S)-Norfluoxetine hydrochloride Form 1        | 1.44                  |
| Starch powder                                 | 226.6                 |
| Silicone Fluid 350 CS                         | 2.0                   |
| Total                                         | 230.04 mg             |

The above ingredients are mixed and filled into hard gelatin capsules in 230.04 mg quantities.

Formulation 2

Capsules are prepared using the following ingredients:

|                                               | Quantity (mg/capsule) |
| --------------------------------------------- | --------------------- |
| (S)-Norfluoxetine hydrochloride Form 3        | 28.78                 |
| Starch powder                                 | 199.2                 |
| Silicone Fluid 350 CS                         | 2.00                  |
| Total                                         | 229.98 mg             |

The above ingredients are mixed and filled into hard gelatin capsules in 229.98 mg quantities.

Formulation 3

Hard gelatin capsules are prepared using the following ingredients:

|                                               | Quantity (mg/capsule) |
| --------------------------------------------- | --------------------- |
| (S)-Norfluoxetine hydrochloride Form 1        | 250                   |
| starch, dried                                 | 200                   |
| magnesium stearate                            | 10                    |
| Total                                         | 460 mg                |

The above ingredients are mixed and filled into hard gelatin capsules in 460 mg quantities.

Formulation 4

A tablet is prepared using the ingredients below:

|  | Quantity (mg/tablet) |
|---|---|
| (S)-Norfluoxetine hydrochloride Form 3 | 250 |
| cellulose, microcrystalline | 400 |
| silicon dioxide, fumed | 10 |
| stearic acid | 5 |
| Total | 665 mg |

The components are blended and compressed to form tablets each weighing 665 mg.

Formulation 5

Tablets each containing 60 mg of active ingredient are made as follows:

| (S)-Norfluoxetine hydrochloride Form 1 | 60 mg |
|---|---|
| starch | 45 mg |
| microcrystalline cellulose | 35 mg |
| polyvinylpyrrolidone (as 10% solution in water) | 4 mg |
| sodium carboxymethyl starch | 4.5 mg |
| magnesium stearate | 0.5 mg |
| talc | 1 mg |
| Total | 150 mg |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

Formulation 6

Capsules each containing 80 mg of medicament are made as follows:

| (S)-Norfluoxetine hydrochloride Form 1 | 80 mg |
|---|---|
| starch | 59 mg |
| microcrystalline cellulose | 59 mg |
| magnesium stearate | 2 mg |
| Total | 200 mg |

The active ingredient, cellulose, starch and magnesium stearate are blended, passed through a No. 45 mesh U.S. sieve, and filled into hard gelatin capsules in 200 mg quantities.

**Claims**

1. Stable, crystalline (S)-norfluoxetine hydrochloride.

11

2. Crystalline (S)-norfluoxetine hydrochloride having substantially the following interplanar spacings in its X-ray powder diffraction pattern:
16.83, 11.89, 10.63, 7.51, 6.05, 5.95, 5.69, 5.60,
5.39, 5.30, 5.13, 4.91, 4.76, 4.67, 4.54, 4.38, 4.20,
4.11, 4.08, 3.96, 3.89, 3.76, 3.69, 3.53, 3.45, 3.36,
3.30, 3.19, 3.12, 3.06, 2.94, 2.77, 2.61, 2.57, 2.49,
2.36, 2.13, 2.06, and 1.62 d (Å).

3. Crystalline (S)-norfluoxetine hydrochloride having substantially the following X-ray powder diffraction pattern:
17.67, 15.65, 12.04, 11.27, 8.86, 7.47, 7.23, 6.01,
5.70, 5.39, 5.22, 4.91, 4.84, 4.55, 4.32, 4.20, 3.94,
3.85, 3.76, 3.71, 3.53, 3.45, 3.30, 3.14, 3.07, 2.94,
2.32, 2.20, and 2.02 d (Å).

4. Crystalline (S)-norfluoxetine hydrochloride having substantially the following X-ray powder diffraction pattern:
15.49, 14.28, 11.36, 9.25, 8.79, 7.18, 6.70, 6.04,
5.92, 5.84, 5.68, 5.58, 5.49, 5.26, 5.19, 4.89, 4.79,
4.64, 4.57, 4.26, 4.17, 4.04, 3.84, 3.78, 3.76, 3.68,
3.60, 3.46, 3.35, 3.30, 3.25, 3.10, 3.02, 2.97, 2.94,
2.84, and 2.60 d (Å).

5. A compound as claimed in any one of Claims 1, 2, or 4 for use in treating a mammal requiring increased neurotransmission of serotonin.

6. A pharmaceutical formulation comprising a compound as claimed in any one of Claims 1, 2, or 4 and one or more pharmaceutically acceptable carriers, diluents or excipients therefor.

7. A process for preparing the compound of Claim 2 which comprises crystallizing (S)-norfluoxetine hydrochloride from either wet ether/petroleum ether, wet ether/heptane, or no more than 15% (v/v) ethyl acetate in heptane.

8. A process for preparing the compound of Claim 3 which comprises crystallizing (S)-norfluoxetine hydrochloride from either tetrahydrofuran/heptane or ethyl acetate/cyclohexane.

9. A process for preparing the compound of Claim 4 which comprises crystallizing (S)-norfluoxetine hydrochloride from either toluene, toluene/heptane or at least 15% (v/v) ethyl acetate in heptane.

**Claims for the following Contracting States : GR and ES**

1. A process for preparing crystalline (S)-norfluoxetine hydrochloride having substantially the following interplanar spacings in its X-ray powder diffraction pattern:
16.83, 11.89, 10.63, 7.51, 6.05, 5.95, 5.69, 5.60,
5.39, 5.30, 5.13, 4.91, 4.76, 4.67, 4.54, 4.38, 4.20,
4.11, 4.08, 3.96, 3.89, 3.76, 3.69, 3.53, 3.45, 3.36,
3.30, 3.19, 3.12, 3.06, 2.94, 2.77, 2.61, 2.57, 2.49,
2.36, 2.13, 2.06, and 1.62 d (Å); which comprises crystallizing (S)-norfluoxetine hydrochloride from either wet ether/petroleum ether, wet ether/heptane, or no more than 15% (v/v) ethyl acetate in heptane.

2. A process for preparing crystalline (S)-norfluoxetine hydrochloride having substantially the following X-ray powder diffraction pattern:
17.67, 15.65, 12.04, 11.27, 8.86, 7.47, 7.23, 6.01,
5.70, 5.39, 5.22, 4.91, 4.84, 4.55, 4.32, 4.20, 3.94,
3.85, 3.76, 3.71, 3.53, 3.45, 3.30, 3.14, 3.07, 2.94,
2.32, 2.20, and 2.02 d (Å); which comprises crystallizing (S)-norfluoxetine hydrochloride from either tetrahydrofuran/heptane or ethyl acetate/cyclohexane.

3. A process for preparing crystalline (S)-norfluoxetine hydrochloride having substantially the following X-ray powder diffraction pattern:
15.49, 14.28, 11.36, 9.25, 8.79, 7.18, 6.70, 6.04,
5.92, 5.84, 5.68, 5.58, 5.49, 5.26, 5.19, 4.89, 4.79,
4.64, 4.57, 4.26, 4.17, 4.04, 3.84, 3.78, 3.76, 3.68,
3.60, 3.46, 3.35, 3.30, 3.25, 3.10, 3.02, 2.97, 2.94,
2.84, and 2.60 d (Å); which comprises crystallizing (S)-norfluoxetine hydrochloride from either toluene, toluene/heptane or at least 15% (v/v) ethyl acetate in heptane.

4. A process for preparing a pharmaceutical formulation which comprises affixing a crystalline (S)-norfluoxetine hydrochloride as defined in Claims 1 or 3 with one or more pharmaceutically-acceptable carriers, diluents, or excipients therefor.

**European Patent Office**

## EUROPEAN SEARCH REPORT

Application Number

EP 91 30 1504

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 868 344 (BROWN)<br>* Column 3, line 18 - column 4, line 21; examples 61,62 * | 1-9 | C 07 C 217/20<br>A 61 K 31/135 |
| P,X | EP-A-0 369 685 (ELI LILLY AND CO.)<br>* Page 6, lines 15-20 * | 1,5,6 | |
| D,A | US-A-4 313 896 (MOLLOY et al.)<br>* Column 8, lines 1-58; example 3 * | 1 | |
| D,A | JOURNAL OF MEDICINAL CHEMISTRY, vol. 31, 1988, pages 1412-1417, American Chemical Society; D.W. ROBERTSON et al.: "Absolute configurations and pharmacological activities of the optical isomers of fluoxetine, a selective serotonin-uptake inhibitor"<br>* Whole article * | 1 | |
| | | | **TECHNICAL FIELDS SEARCHED (Int. Cl.5)**<br><br>C 07 C 213/00<br>C 07 C 217/00 |

The present search report has been drawn up for all claims

| Place of search | Date of completion of the search | Examiner |
|---|---|---|
| THE HAGUE | 30-05-1991 | WELLS A.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document